# EUROPÄISCHE PATENTSCHRIFT

(11) **EP 0 831 152 B1**
(45) Veröffentlichungstag und Bekanntmachung des Hinweises auf die Patenterteilung: **29.05.2002**
(21) Anmeldenummer: 97250279.3
(22) Anmeldetag: 17.09.1997
(51) Int. Cl.: C12Q 1/18, G01N 33/48

(54) **Verfahren und Vorrichtung zur Bestimmung der Nitrifikanten-Toxizität**
Process and apparatus for determining toxicity to nitrifying bacteria
Procédé et dispositif de détermination de la toxicité de bactéries nitrifiantes

(30) Priorität: 19.09.1996 DE 19640334
(43) Veröffentlichungstag der Anmeldung: 25.03.1998
(73) Patentinhaber: LAR Analytik und Umweltmesstechnik GmbH, D-10963 Berlin (DE)
(72) Erfinder: Pilz, Ulrich, Dr.-Ing., 13465 Berlin (DE)
(74) Vertreter: Heinze, Ekkehard, Dipl.-Phys. Dr.

(56) Entgegenhaltungen:
- DE-A- 2 513 650
- DE-A- 2 951 707
- Chem. abstr., Band 108, Nr. 11, 14. M rz 1988 (Columbus, Ohio, USA), Seite 389, Zusammenfassungs- -Nr. 100512k, ROBINSON, R.J. 'Assessment of methods for evaluating the treatability of sewage and the effects of industrial discharges'; & Toxic.Assess. 1988, 3(1), 17-31.

## Beschreibung

Die Erfindung betrifft ein Verfahren zur Bestimmung der Nitrifikaten-Toxizität einer wäßrigen Lösung (speziell aufzubereitendem Wasser bzw. Abwasser) und eine Vorrichtung zur Durchführung des Verfahrens.

Die Analyse von Wasser bzw. Abwasser hinsichtlich seiner Inhaltsstoffe und der bei der Aufbereitung/Entsorgung eingesetzten Hilfsmittel hinsichtlich ihrer spezifischen Leistungsfähigkeit sind unabdingbare Voraussetzungen für eine den hohen aktuellen Qualitätsanforderungen genügende und effiziente Wasseraufbereitung und Abwasserentsorgung.

Bei der Klärung von Abwässern aus dem privaten und gewerblichen Bereich stellt der (biologische) Abbau organischer Kohlenstoffverbindungen ein vordringliches Ziel dar.

Aus DE 29 51 707 C2 ist eine Einrichtung zur Bestimmung der Konzentration von biologisch abbaubaren Stoffen in Abwässern bekannt, mit der der biologische Sauerstoffbedarf des Abwassers (als Maß für diese Konzentration) über eine Vergleichsmessung der Sauerstoffkonzentrationsdifferenz am Ein- und Ausgang zweier parallel betriebener Reaktoren ermittelt wird.

Im Ergebnis des Abbaus der Kohlenstoffverbindungen, insbesondere als Folgeprodukt der bakteriellen Verwertung von Eiweißstoffen, bilden sich Stickstoffverbindungen (Ammonium), die in nachteiliger Weise eine Eutrophierung der Gewässer bewirken und eine Gefahr für den Fischbesatz darstellen. Diese Folgeprodukte sind daher im Rahmen der Abwasserreinigung ebenfalls zu entfernen. Die Entfernung des Ammoniums läuft in zwei Stufen ab: Zunächst wird Ammonium unter Veratmung von im Wasser gelöstem Sauerstoff ("aerob") durch spezielle Mikroorganismen ("Nitrifikanten") zu Nitrit und Nitrat oxidiert, und anschließend erfolgt unter Sauerstoffmangelbedingungen (quasi "anaerob") durch andere Mikroorganismen eine Reduktion des Nitrats ("Denitrifikation") zu gasförmigem Stickstoff, der ohne weiteres in die Atmosphäre entweichen kann.

Die an dem Nitrifikationsprozeß beteiligten Bakterien (Nitrifikanten) - der Gattungen Nitrobacter und Nitrosomonas - sind sehr schadstoffempfindlich. Eine ganze Reihe von relativ häufig in Abwässern auftretenden Schadstoffen setzt die Vitalität dieser Bakterien signifikant herab und beeinflußt daher entscheidend die Nitrifikationsleistung, so daß die Erfassung von deren toxischer Wirkung (kurz zu bezeichnen als "Nitrifikanten-Toxizität") höchst relevant für den Betrieb von Kläranlagen ist. Hieraus ergibt sich der Bedarf nach einem Verfahren und einer Vorrichtung zu dessen Durchführung, mit denen die Nitrifikanten-Toxizität eines Wassers bzw. Abwassers meßtechnisch quasi-kontinuierlich erfaßt werden kann.

Die Erfassung und auch Gehaltsbestimmung toxischer Substanzen in Abwasser oder anderen wäßrigen Lösungen unter Nutzung von Mikroorganismen als Testorganismen ist in vielen Abwandlungen bekannt; die bekannten Verfahren sind jedoch größtenteils als Verfahren für die Analytik einzelner Proben im Labor konzipiert und nicht ohne weiteres für die Online-Prozeßüberwachung und -steuerung geeignet.

Ein kontinuierlich arbeitendes, gattungsgemäßes Verfahren und Gerät sind aus M. Gründel: "Kontinuierliche On-line-Analytik eines Nitrifikantentoximeters", WAP 4/95, S. 53, bekannt. Dieses Verfahren und Gerät sind hinsichtlich der Genauigkeit und Reproduzierbarkeit der Meßergebnisse noch verbesserungsbedürftig.

Der Erfindung liegt die Aufgabe zugrunde, ein kontinierliches Verfahren und eine entsprechende Vorrichtung zur Bestimmung der Nitrifikaten-Toxizität mit verbesserter Genauigkeit und Reproduzierbarkeit der Ergebnisse anzugeben.

Die Aufgabe wird hinsichtlich des Verfahrens durch ein Verfahren mit den Merkmalen des Anspruchs 1 und hinsichtlich der Vorrichtung durch eine Vorrichtung mit den Merkmalen des Anspruchs 13 gelöst.

Die Erfindung schließt den Gedanken ein, für den Meßvorgang streng reproduzierbare Verhältnisse zu schaffen und hierzu insbesondere das an der Probe erhaltene Meßergebnis über eine Referenzmessung an einer definitiv unbelasteten Referenzprobe zu normieren, wobei die Proben- und die Referenzmessung unter exakt definierten Bedingungen stattfinden und diese mit minimalem Aufwand gewährleistet werden.

Wichtig hierfür sind speziell eine hinreichende Spülung der Meßzelle zwischen den Messungen und insbesondere vor den Referenzmessungen, um das verfälschende Vorhandensein von Resten der Vorprobe in der Meßzelle auszuschließen, sowie präzise automatisch gesteuerte Abläufe bzw. Dosierungen bei der Züchtung der Bakterienkultur und deren Zuführung zur Meßzelle.

In zwei alternativen Ausprägungen des erfindungsgemäßen Verfahrens- und Vorrichtungsgedankens wird mit ein und derselben Meßzelle für die Proben- und Referenzmessungen oder mit zwei getrennten, verfahrenstechnisch übereinstimmenden Meßzellen für beide Messungsarten gearbeitet. Die erste Alternative ist gerätetechnisch weniger aufwendig, führt jedoch, falls häufige Referenzmessungen nötig werden, zu einer Verringerung der Zeitnähe des Verfahrens. Die zweite Alternative ist aufwendiger, aber wegen der grundsätzlichen Eliminierung eines Einflusss belasteter Proben auf die Referenzmessungen potentiell genauer, und auch häufige Referenzmessungen sind ohne nachteiligen Einflüß auf die On-line-Charakteristik des Verfahrens ohne weiteres möglich.

Im allgemeinen wird jedoch (in beiden Fällen) der zeitliche Abstand der Referenzmessungen wesentlich größer sein als derjenige der laufenden Messungen an der zu untersuchenden Lösung (Wasser oder Abwasser), wobei der letztere durch die Summe der Zeitspannen zum Spülen der Meßzelle(n), Befüllen mit Proben- bzw. Referenzflüssigkeit sowie Bakterienkultur und Umsetzen/Messen bestimmt ist.

Die eigentliche Auswertung zur Bestimmung der Nitrifikanten-Toxizität erfolgt anhand eines Vergleichs des Abfallsgradienten des Sauerstoffgehaltes als Funktion der Zeit in der Probenlösung während eines Abschnitts des vierten Zeitintervalls mit dem Abfallsgradienten in reinem Wasser.

Vor Ausführung dieses Vergleiches ist bei höheren Genauigkeitsanforderungen der Proben-Abfallsgradient dahingehend zu korrigieren, daß die Sauerstoffzehrung infolge reiner Substratoxidation (also ohne Nitrifikationsanteile) auskorrigiert wird. Dies erfolgt, indem jeweils in vorgegebenen Zeitabständen zwischen dem ersten und dem nachfolgenden dritten Zeitintervall in einem fünften Zeitintervall eine Messung der Zeitabhängigkeit der Sauerstoffkonzentration in der wäßrigen Lösung ohne Nitrifikanten-Bakterien zur Gewinnung eines Korrekturwertes durchgeführt wird. Speziell dient der Abfallsgradient des Sauerstoffgehaltes als Funktion der Zeit während eines Abschnittes des fünften Zeitintervalls als subtraktiver Korrekturwert für den im vierten Zeitintervall (in Anwesenheit der Nitrifikanten in der Probe) ermittelten Abfallsgradienten.

Das Meßverfahren - vorzugsweise auch die Züchtung der Mikroorganismenkultur - wird zweckmäßigerweise bei einer im wesentlichen konstantgehaltenen Temperatur im für eine Nitrifikation günstigen Bereich zwischen 20°C und 40°C, bevorzugt zwischen 25°C und 30°C, durchgeführt.

Eine Reihe von die Genauigkeit und Reproduzierbarkeit der Messungen wesentlich beeinflussenden Maßnahmen bezieht sich auf die zum Verfahren gehörende kontinuierliche Züchtung der Nitrifikanten-Bakterienkultur in einem von der Probe getrennten und daher die Kultur prinzipiell vor toxischen Einflüssen schützenden Kulturgefäß (Fermenter):

Zum einen wird zur Sicherung einer gleichbleibend hohen Qualität der Kultur in dem Kulturgefäß die Bakteriendichte der Nitrifikanten-Bakterienkultur und das Gesamtvolumen aus Nährlösung und Bakterienkultur jeweils auf einen konstanten Wert geregelt. Dies umfaßt die gesteuerte Zuführung von Nährlösung und die Ableitung überschüssiger Mischung aus Kultur und Nährlösung. Die Steuerung der Zudosierung von Nährlösung erfolgt in zweckmäßiger Weise derart, daß in dem Kulturgefäß kontinuierlich die Lichtstreuung und hierdurch die Trübung gemessen und in Abhängigkeit vom Lichtstreu-Meßwert (Trübungswert) wahlweise gesteuert frische Nährlösung dem Kulturgefäß zugeführt wird. Ein dem zugeführten Nährlösungsvolumen gleiches Volumen an Nährlösungs-Kultur-Mischung wird in einfacher Weise über einen Überlaufrand aus dem Kulturgefäß abgeleitet.

Weiterhin wird in dem Kulturgefäß - kontinuierlich oder periodisch in kurzen Abständen - der pH-Wert gemessen und in Abhängigkeit vom pH-Meßwert wahlweise gesteuert eine Base dem Kulturgefäß zugeführt, um den pH-Wert, vorzugsweise bei einem Wert im Bereich zwischen 7,0 und 8,5, konstantzuhalten.

Als Nährlösung wird bevorzugt eine wäßrige Lösung mineralischer Komponenten mit einem Ammoniumsalz und/oder Harnstoff als Stickstoffquelle und Hydrogencarbonat als Kohlenstoffquelle eingesetzt, die als Selektivnährlösung für ausgewählte Nitrifikanten-Bakterien der Gattungen Nitrobacter und/oder Nitrosomonas wirkt.

Auf die skizzierte Weise kann die Kultur mit stabiler Qualität unter unsterilen Bedingungen bereitgestellt werden, was mit relativ geringem Kulturaufwand die Gewährleistung exakt reproduzierbarer Sauerstoffzehrungsraten ermöglicht.

Insgesamt ermöglicht das Verfahren gegenüber bekannten Verfahren der mirobiellen Toxizitätserfassung - etwa unter Einsatz von Pseudomonas- oder Photobakterium phosphoreum-Kulturen - eine deutliche Erniedrigung der Nachweisgrenze für bestimmte Schadstoffe, beispielsweisse für Cyan-, Quecksilber - und Kupferverbindungen und bestimmte organische Schadstoffe.

Eine Vorrichtung zur Durchführung des Verfahrens zeichnet sich durch eine mit einem Zeitgeber verbundene mehrkanalige Ablaufsteuerung aus, die jeweils nach Ablauf vorbestimmter Zeitintervalle Steuersignale an Stellmittel zum selbsttätitigen Öffnen bzw. Schließen ausgewählter mit der Meßzelle bzw. Den Meßzellen verbundenen Leitungen bzw. an in diesen Leitungen angeordnete Fördermittel für die verschiedeneen Einsatzstoffe (Nährlösung, pH-Wert-Regulans, Wasser, ggfs. Reinigungslösung) zu deren Aktivierung sowie an eine Sauerstoffgehalts-Auswertungseinrichtung ausgibt. Der Zeitgeber, die Ablaufsteuerung und die Auswertungseinrichtung sind in praktisch zweckmäßiger Weise integriert und in Mikroprozessortechnik ausgeführt.

Im Kulturgefäß sind in vorteilhafter Weise eine Anordnung zur Erfassung der Lichtstreuung und ein pH-Wert-Sensor angeordnet, die mit einer Auswertungseinrichtung für die züchtungsrelevanten Bedingungen in der Kultur-Nährlösungs-Mischung verbunden sind, welche ihrerseits mit einem Dateneingang der Ablaufsteuerung verbunden ist und dieser Datensignale zuführt, die in Steuersignale für ein Nährlösungs-Absperrventil und/oder eine Nährlösungs-Förder-bzw. Dosierpumpe und Absperrventil und/oder eine Fördereinrichtung für das pH-Regulans umgesetzt werden.

An der Zuflußleitung zur Meßzelle sind - mit dieser mittels durch Steuersignale der Ablaufsteuerung betätigter Absperrventile jeweils wahlweise verbindbar - ein Probenahmeröhrchen, welches in eine das zu untersuchende Wasser bzw. Abwasser führende Leitung ragt, ein Wasserleitungsanschluß und ein Reinigungslösungsbehälter vorgesehen. Weiterhin ist an der Zuflußleitung zur Meßzelle ein Einlaßventil oder eine Pumpe zur Einspeisung von Luft oder Sauerstoff vorgesehen, das/die vorzugsweise durch ein Steuersignal der Ablaufsteuerung wahlweise zu öffnen bzw. zu aktivieren ist.

Das vorliegende Verfahren und die angegebene Vorrichtung eignen sich besonders für die Online-Prozeßüberwachung und -steuerung wasser- und abwassertechnischer Systeme sowie für weitere laufend und bevorzugt automatisch auszuführende Umweltüberwachungs- und Steuerungsaufgaben, etwa die Überwachung von Bodenwaschanlagen oder von Deponien hinsichtlich der Dichtigkeit von Deponieabdeckungen und die Ausführung von ggfs. erforderlichen Maßnahmen zur Gefahrabwendung. Die Auswertungseinrichtung der Vorrichtung ist für den Einsatz in solchen Systemen vorteilhaft direkt mit deren Prozeßsteuerung verbunden, so daß anhand der Toxizitätsbefunde über geeignete Stellmittel des Systems unmittelbar eine Drosselung oder Sperrung von Zuläufen, eine Umleitung oder Zwischenspeicherung von hochbelastetem Abwasser o.ä. Maßnahmen veranlaßt werden können.

Weitere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein schematisches Blockschaltbild einer Vorrichtung zur Durchführung des Verfahrens nach einer ersten Ausführungsform,
- Figur 2: ein weiteres Blockschaltbild einer weiteren Vorrichtung zur Durchführung des Verfahrens und
- Figur 3: eine Detaildarstellung zu Fig. 1 in Form eines Funktions-Blockschaltbildes der Auswertungseinheit.

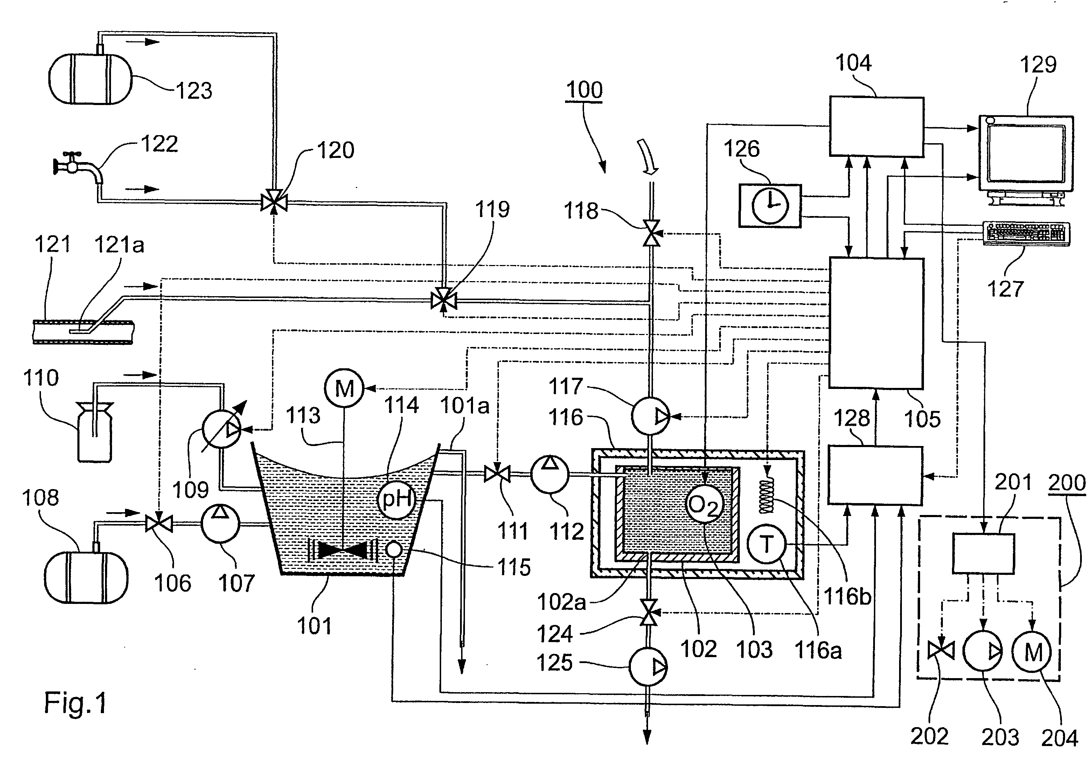

Fig. 1 zeigt eine Vorrichtung 100 zur Bestimmung der Nitrifikanten-Toxizität in Verbindung mit einer - hier nur symbolartig dargestellten - Prozeßsteuerung einer Wasserversorgungs- oder Abwasserentsorgungsanlage 200. Hauptbestandteile der Meßvorrichtung sind ein Fermenter 101, eine geschlossene Meßzelle 102 mit Gelöstsauerstoffühler 103, eine mit diesem verbundene Auswertungseinheit 104 und ein Mikrocontroller 105 als Ablaufsteuereinheit.

Mit dem Fermenter (Kulturgefäß) 101, der ein Volumen von ca. 1-2 1 aufweist, sind zulaufseitig über ein Absperrventil 106 und eine Förderpumpe 107 ein Nährlösungsbehälter 108 sowie über eine Dosierpumpe 109 ein Vorratsbehälter 110 für Natronlauge verbunden. Der Nährlösungsbehälter 108 enthält eine als Selektivnährlösung für Nitrifikanten-Bakterien geeignete wäßrige Lösung, die Ammoniumsalze und Harnstoff als Stickstoffquellen und Hydrogencarbonat als Kohlenstoffquelle in geeigneter Konzentration aufweist. Ablaufseitig ist der Fermenter über ein Absperrventil 111 und eine Förderpumpe 112 mit der Meßzelle 102 verbunden; zudem weist er einen Überlaufrand mit Ablauf 101a auf, über den überschüssiges Kultur-Nährlösungs-Gemisch selbsttätig ausgetragen und verworfen wird. Der Fermenter ist mit einem Rührwerk 113, einem ISFET-pH-Sensor 114 und einem Streulichtfühler 115 ausgestattet.

Die Meßzelle 102, die einen Rauminhalt von einigen Millilitern hat, ist bei der hier gezeigten Ausführung in einem Thermostaten 116 mit Temperaturfühler 116a und T-Regeleinrichtung 116b untergebracht. Sie ist zulaufseitig - neben dem Fermenter - über eine Förderpumpe 117 und ein Lufteinströmventil 118 sowie ein erstes Dreiwege-Absperrventil 119 und ein zweites Dreiwege-Absperrventil 120 mit einem in ein Abwasserrohr 121 ragenden Probenahmeröhrchen 121a, einer Wasserleitung 122 und einem Behälter 123 für Reinigungslösung verbunden. Sie weist einen mit einem Absperrventil 124 versehenen Ablauf 102a auf, an den eine Absaugpumpe 125 angeschlossen ist.

Der Mikrocontroller 105 und die Auswertungseinheit 104 sind über einen Steuereingang mit einer gemeinsamen Zeitbasis 126 und einer Eingabetastatur 127 verbunden, die Auswertungseinheit darüber hinaus mit einem Steuerausgang des Mikrocontrollers. Letzterer ist eingangsseitig mi einer Prozeßgrößen-Auswertungseinheit 128 verbunden, die ihrerseits eingangsseitig mit dem pH-Sensor 114, dem Trübungsmesser 115 und dem T-Fühler 116a verbunden ist. Ausgangsseitig sind Mikrocontroller 105 und Auswertungseinheit 104 mit einem Bildschirm 129 zur Darstellung der Meßprozeßdaten und der Auswertungsergebnisse verbunden. Der Mikrocontroller ist ausgangsseitig mit den Absperrventilen und Pumpen sowie dem Motor des Rührwerks 113 und der T-Regelung 116b verbunden, die Auswertungseinheit 104 mit einer Prozeßsteuereinheit 201 des Wasser-Wasser-/Abwassersystems 200, die ihrerseits über Steuerleitungen mit einem Absperrventil 202, einer Förderpumpe 203 sowie einem Stellmotor 204 verbunden gezeigt ist. Durch Betätigung derartiger Stellmittel kann beispielsweise bei Ermittlung einer erhöhten Nitrifikanten-Toxizität des in der Leitung 121 geführten Abwassers automatisch die Abwasserzuleitung zu einem Klärbecken abgesperrt, dem belasteten Abwasser zur Verdünnung sauberes Wasser zugefördert oder ein chemisches Neutralisierungsmittel zudosiert werden, um Beeinträchtigungen eines biologischen Klärprozesses zu vermeiden oder zumindest zu minimieren.

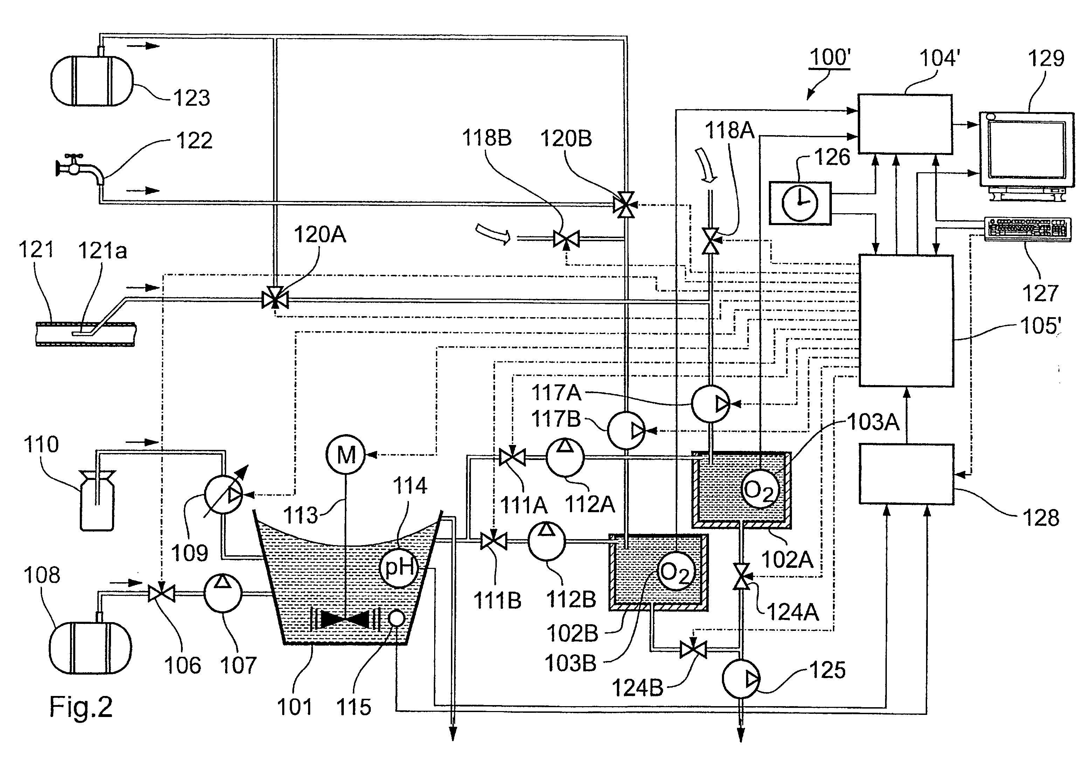

Der Ablauf des Kultur- und Meßverfahrens in der in Fig. 2 gezeigten Vorrichtung ist in einer bevorzugten Ausführung wie folgt:

Im Kulturgefäß 101 wird kontinuierlich eine Mischkultur aus Bakterien der Gattungen Nitrobacter und Nitrosomonas in der oben spezifizierten Nährlösung bei einem pH-Wert von 8,0 kultiviert, wobei die Mikroorganismendichte kontinuierlich durch Trübungsmessung mittels der Streulichtsensoranordnung 115 und der pH-Wert mittels des pH-Sensors 114 erfaßt wird. Da die Nitrifikation in der Nährlösung mit einer Säurebildung verbunden ist, erhöht sich ständig tendenziell der pH-Wert im Kulturgefäß. Wird durch die Prozeßdaten-Auswertungseinheit 128 aufgrund des Sensorsignals eine über einer bestimmten Schwelle liegende Erhöhung des pH-Wertes ermittelt, wird ein die pH-Sollwertüberschreitung widerspiegelndes Signal an den Controller 105 ausgegeben, der seinerseits ein Ansteuersignal zur Förderung einer vorbestimmten Menge von Natronlauge aus dem Behälter 110 an die Dosierpumpe 109 ausgibt. Ähnlich erfolgt bei Überschreitung eines vorbestimmten Trübungsgrades der Mischung im Kulturgefäß als Folge einer Überschreitung der vorbestimmten Mikroorganismendichte aufgrund des entsprechendne Signals des Streulichtsensors 115 die Ausgabe eines Datensignals durch die Auswertungseinheit an den Controller, woraufhin dieser das Absperrventil 106 für eine aus dem Datensignal berechnete Zeit öffnet und dadurch die Förderung einer vorbestimmten Menge von Nährlösung aus dem Vorratsbehälter 108 in den Fermenter 101 bewirkt. Gleichzeitig wird eine der zugeführten Nährlösungsmenge entsprechende Menge der Mischung über den Überlaufrand 101a ausgepült. Die zugeführte Nährlösung verdünnt die Mischung und führt die Bakteriendichte auf den gewünschten Wert zurück, woraufhin der Trübungsgrad wieder absinkt.

Das beschriebene Vorgehen übt in vorteilhafter Weise einen Selektionsdruck auf die Kultur aus, da die am schnellsten wachsenden Bakterien die Dosierfrequenz bestimmen und langsamer wachsende Spezies tendenziell ausgespült werden. Damit steht permanent eine konstant leistungsfähige Kultur mit hoher Vitalität für die eigentliche Messung bereit.

Bevor diese ausgeführt wird, wird die Meßzelle 102 (die in größeren Abständen, z.B. einmal täglich, mit Reinigungslösung aus dem Behälter 123 einer Grundreinigung unterzogen wird) jedesmal durch Öffnen der Verbindung zum Probenahmeröhrchen 121a über Betätigung des Ventils 119 sowie des Ablaufes durch Öffnen des Ventils 124 und Betätigung der Pumpe 125 mit ca. 200 ml Probelösung gespült. Dann wird das Ablaufventil 124 geschlossen und die Pumpe 125 gestoppt und in der verbleibendenn Spanne der Öffnungsdauer des Ventils 119 (des ersten Zeitintervalls; insgesamt ca. 4 min) die Meßzelle über die Pumpe 117 mit über das geöffnete Ventil 118 belüftetem Probe-Abwasser gefüllt. Anschließend aktiviert der Controller 105 die Sauerstoffgehalts-Auwertungseinheit 104, und für eine Zeitspanne von ca. 2 min (fünftes Zeitintervall) wird die sogenannte Grundzehrung, d.h. der Sauerstoffgehaltsabfall ohne Bakterienkultur, ermittelt. Die Meßkurve und/oder der Abfallsgradient in einem vorbestimmten Bereich der Meßkurve wird gespeichert.

Anschließend gibt der Controller ein Steuersignal zum Öffnen der Verbindungsleitung zwischen Fermenter 101 und Meßzelle 102 während eines vorbestimmten Intervalls (drittes Zeitintervall; ca. 2 min) an das Ventil 111, und die Pumpe 112 transportiert eine über die Intervalldauer exakt vorbestimmte Menge Nitrifikantensuspension in die Meßzelle. Dem schließt sich eine zweite Meßphase von ca. 8 min (viertes Zeitintervall) an, während dessen der Sauerstoffgehaltsabfall mit Bakterienkultur erfaßt wird. Auch diese Meßkurve und oder der Abfallsgradient im vorbestimmten Bereich der Kurve wird gespeichert.

Anschließend werden das Ablaufventil 124 geöffnet, die Absaugpumpe 125 eingeschaltet und das Dreiwegeventil 119 auf den Frischwasserzulauf 122 umgeschaltet. Die Meßzelle wird zunächst mit einigen hundert Millilitern reinem Wasser gespült und dann mit diesem befüllt; diese Phase (zweites Zeitintervall) nimmt ca. 5-6 min in Anspruch. Anschließend erfolgt unter Einhaltung des dritten Zeitintervalls (hier 2 min) eine dosierte Zuführung von Nitrifikantensuspension und während des vierten Zeitintervalls (hier 8 min) eine Erfassung des Sauerstoffgehaltsabfalls der Suspension in reinem Wasser. Die erhaltene Meßkurve und/oder der entsprechende Abfallsgradient wird wieder gespeichert.

Aus den bei den obigen Schritten gespeicherten Meßkurven bzw. Gradientenwerten wird - wie weiter unten etwas näher erläutert - schließlich die Nitrifikationshemmung im untersuchten Abwasser und hieraus dessen Nitrifikanten-Toxizität ermittelt. Anzumerken ist, daß der oben beschriebene Ablauf einer alternierenden Messung an Proben-Abwasser und einer Referenzmessung an reinem Wasser in der Praxis lediglich in größeren Zeitabständen gesteuert wird; über den weitaus größten Teil der Betriebszeit der Meßanordnung werden nur aufeinanderfolgende Proben-Messung durchgeführt.

Fig. 2 zeigt eine gegenüber Fig. 1 modifizierte Meßanordnung 100', die allerdings wesentliche Komponenten mit der oben beschriebenen Anordnung gemein hat. Diese sind mit denselben Bezugsziffern wie in Fig. 1 bezeichnet und werden jetzt nicht nochmals beschrieben. Eine Verbindung zur Steuerung eines Wasser- oder Abwasserbehandlungssystems ist hier nicht gezeigt, kann aber bestehen.

Der wesentliche Unterschied gegenüber der oben beschriebenen Anordnung besteht im Vorsehen zweier Meßzellen 102A und 102B gleicher Geometrie mit jeweils einem Sauerstoffsensor 103A bzw. 103B und separaten (hier nihct gesondert bezeichneten) Abläufen, von denen die Meßzelle 102A über ein Dreiwegeventil 120A und eine Pumpe 117A - abgesehen von der Verbindung zum Reinigungslösungsbehälter 123 - ausschließlich mit der abwasserführenden Leitung 121, die Zelle 102B hingegen über ein Dreiwegeventil 120B und eine Pumpe 117B nur mit der Frischwasserzufuhr 122 verbunden ist. Die Zuleitungen beider Zellen weisen einen Lufteinlaß mit Absperrventil 118A bzw. 118B auf. Die Meßzellen sind hier nicht thermostatiert. Die Auswertungseinheit 104' und der Controller 105' sind in Anzahl und Belegung der Ein- und Ausgänge und ihren Funktionen dem modifizierten Aufbau der Anordnung angepaßt.

Der zeitliche Ablauf der Meßvorgänge - einschließlich der vorbereitenden Spülung vor der Proben-Messung - entspricht dem oben beschriebenen, die Referenzmessungen an reinem Wasser werden jedoch nicht zwischen Proben-Messungen in ein und derselben Meßzelle, sondern zeitlich und räumlich unabhängig von diesen ausgeführt. Die Spülzeiten in der Referenz-Meßzelle 102B können entsprechend verkürzt werden, und die quasi-kontinuierliche Abwasserkontrolle wird zu keinen Zeitpunkt durch Referenzmessungen unterbrochen.

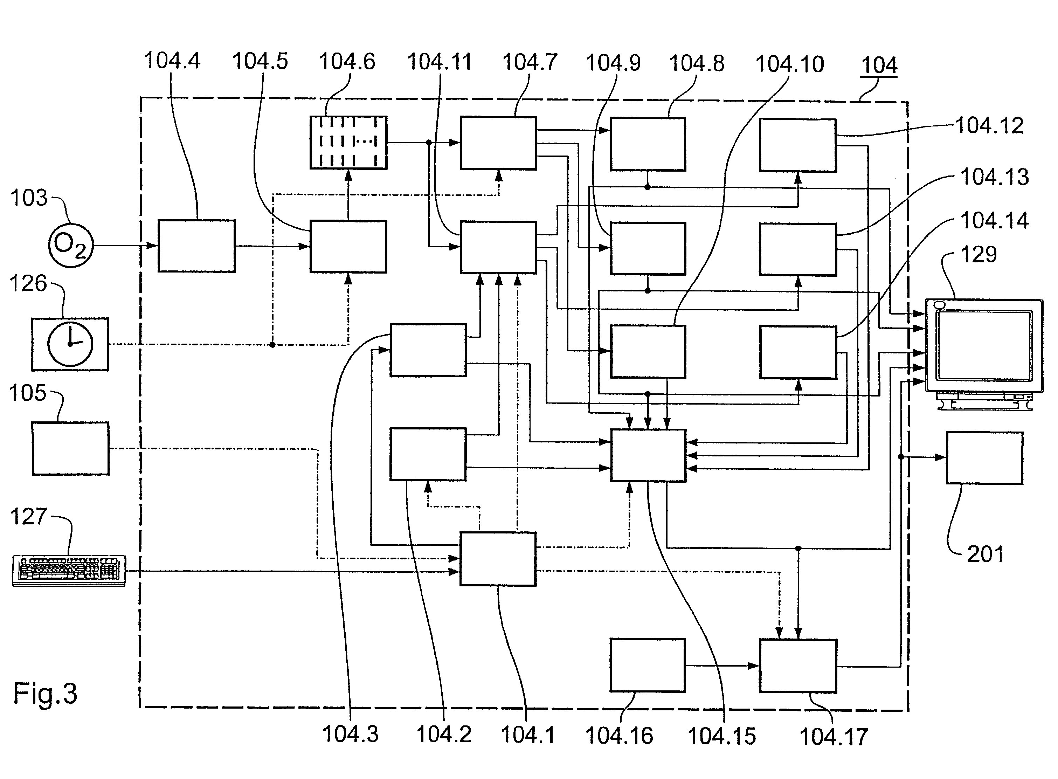

Fig. 3 zeigt - in stark vereinfachter Darstellung - ein Funktions-Blockschaltbild einer Ausführungsform der Auswertungseinheit 104 nach Fig. 1.

Die Auswertungseinheit umfaßt eine interne Auswertungssteuereinheit 104.1, die eingangsseitig mit dem Controller 105 und der Tastatur 127 verbunden ist, sowie einen mit der internen Steuereinheit verbundenen Programmspeicher 104.2 und Arbeitsspeicher 104.3. Der Sauerstoffsensor 103 ist mit einer Signalaufbereitungseinheit 104.4 verbunden, die das Sensorsignal aufbereitet und eine A/D-Umsetzung ausführt, so daß es am Ausgang störbefreit in einem für die digitale Weiterverarbeitung geeigneten Format vorliegt. Über eine vom Zeitgeber 126 gesteuerte Speichersteuerung 104.5 wird das Meßsignal einer Messung in einen Zwischenspeicher 104.6 abgelegt.

Nach Abschluß jeder Messung wird das zwischengespeicherte Meßergebnis (jeweils eine Meßkurve der Sauerstoffkonzentration in Abhängigkeit von der Zeit) über eine - wiederum durch den Zeitgeber gesteuerte - Ablage-Speichersteuerung 104.7 in einen von drei Meßkurvenspeichern 104.9 bis 104.10 übernommen - je nachdem, ob es sich um die Meßkurve für die Grundzehrung in einer Probe, für die Gesamtzehrung in einer Probe oder für die Zehrung in reinem Wasser handelt. Zugleich wird sie in eine Gradienten-Berechnungseinheit 104.11 übernommen, die anhand eines im Programmspeicher 104.2 gespeicherten Berechnungsprogramms und von im Arbeitsspeicher 104.2 gespeicherten Randwerten einen Gradienten des Sauerstoffgehaltsabfalls für die Meßkurve berechnet. Der berechnete Wert wird - je nachdem, um welche Art Messung es sich handelte - in einen von drei Gradientenwertspeichern 104.12, 104.13 oder 104.1 übernommen.

Wiederum unter Zugriff auf in den Speichern 104.2, 104.3 gespeicherte Daten und Algorithmen, wird in einer arithmetischen Berechnungseinheit 104.15 aus den gespeicherten Gradientenwerten und ggfs. Meßkurvenverläufen der Einfluß des untersuchten Abwassers auf die Nitrifikations-Sauerstoffzehrung berechnet, und im Vergleich mit gespeicherten Daten aus einem Vergleichswertspeicher 104.16 kann in einer mehrstufigen Vergleicher- und Bewertungseinheit 104.17 letztlich eine quantitative Aussage zur Nitrifikanten-Toxizität des Abwassers abgeleitet werden, die einem Eingang der Prozeßsteuereinheit 201 zugeführt werden kann. Die Ausgänge der Speicher 104.8 bis 104.10 und der Einheiten 104.15 und 104.17 sind zudem mit dem Bidschirm verbunden, so daß die Meßkurven und wichtigsten Berechnungsergebnisse auch für den Bediener dargestellt werden können. Selbstverständlich ist auch eine Ausgabe über eine Datenschnittstelle, Drucker oder Plotter sowie eine Langfristspeicherung auf Datenträger möglich.

Die Erfindung beschränkt sich in ihrer Ausführung nicht auf die vorstehend angegebenen bevorzugten Ausführungsbeispiele. Vielmehr ist im durch die Ansprüche gesteckten Rahmen eine Anzahl von Varianten denkbar, welche von der dargestellten Lösung auch bei anders gearteten Ausführungen Gebrauch machen.

## Patentansprüche

1. Verfahren zur Bestimmung der Nitrifikanten-Toxizität einer wäßrigen Lösung, insbesondere von Wasser oder Abwasser, durch Bestimmung der Zeitabhängigkeit des Sauerstoffgehalts einer mit der vorab belüfteten wäßrigen Lösung vermischten Nitrifikanten-Bakterienkultur in einer geschlossenen Meßzelle, wobei die Züchtung der Nitrifikanten-Bakterienkultur in einer Nährlösung in einem separaten Kulturgefäß einen Teil des Verfahrens bildet, **dadurch gekennzeichnet, daß**
- ein Zufluß der Meßzelle periodisch für ein erstes Zeitintervall mit einer die wäßrige Lösung führenden Leitung und nach einer vorbestimmten Anzahl erster Zeitintervalle oder zu vorbestimmten Zeitpunkten für ein zweites Zeitintervall mit einer reines Wasser führenden Leitung verbunden wird, wobei zwischen den Zeitintervallen die Meßzelle über einen Abfluß entleert und mindestens für den größeren Teil des ersten Zeitintervalls der Abfluß geöffnet gehalten wird, um die Meßzelle zu spülen, und anschließend die Meßzelle bis zu einem vorgegebenen Pegel oder mit einer vorgegebenen Menge belüfteter wäßriger Lösung bzw. belüfteten reinen Wassers befüllt wird,
- die Meßzelle nach dem Befüllen mit wäßriger Lösung oder reinem Wasser jeweils für ein drittes Zeitintervall mit dem Kulturgefäß verbunden und ihr eine vorbestimmte Menge der Nitrifikanten-Bakterienkultur zugeführt wird und
- nach jedem dritten Zeitintervall in der Meßzelle bei geschlossenem Zu- und Abfluß jeweils für ein viertes Zeitintervall abwechselnd an Mischungen wäßriger Lösung oder reinen Wassers mit der Nitrifikanten-Bakterienkultur eine Messung der Zeitabhängigkeit der Sauerstoffkonzentration durchgeführt wird.

2. Verfahren zur Bestimmung der Nitrifikanten-Toxizität einer wäßrigen Lösung, insbesondere von Wasser oder Abwasser, durch Bestimmung der Zeitabhängigkeit des Sauerstoffgehalts einer mit der vorab belüfteten wäßrigen Lösung vermischten Nitrifikanten-Bakterienkultur in einer geschlossenen Meßzelle, wobei die Züchtung der Nitrifikanten-Bakterienkultur in einem separaten Kulturgefäß einen Teil des Verfahrens bildet, **dadurch gekennzeichnet, daß**
- periodisch für jeweils ein erstes Zeitintervall ein Zufluß einer ersten Meßzelle mit einer die wäßrige Lösung führenden Leitung und für ein zweites Zeitintervall ein Zufluß einer zweiten, mit der ersten Meßzelle verfahrenstechnisch identischen Meßzelle mit einer reines Wasser führenden Leitung verbunden wird, wobei zwischen den ersten und zweiten Zeitintervallen die Meßzellen über einen Abfluß entleert werden und mindestens für den größeren Teil des ersten Zeitintervalls der Abfluß der ersten Meßzelle geöffnet gehalten wird, um die erste Meßzelle zu spülen, und anschließend die Meßzellen bis zu einem vorgegebenen, gleichen Pegel oder mit einer vorgegebenen, gleichen Menge belüfteter wäßriger Lösung bzw. belüfteten reinen Wassers befüllt werden,
- die Meßzellen nach dem Befüllen mit wäßriger Lösung oder reinem Wasser jeweils für ein drittes Zeitintervall mit dem Kulturgefäß verbunden werden und ihnen eine vorbestimmte, gleiche Menge der Nitrifikanten-Bakterienkultur zugeführt wird und
- nach dem dritten Zeitintervall in den Meßzellen bei geschlossenem Zu- und Abfluß jeweils für ein viertes Zeitintervall an der Mischung wäßriger Lösung bzw. reinen Wassers mit der Nitrifikanten-Bakterienkultur jeweils eine Messung der Zeitabhängigkeit der Sauerstoffkonzentration durchgeführt wird.

3. Verfahren nach Anspruch 2, **dadurch gekennzeichnet, daß** aufeinanderfolgende zweite Zeitintervalle einen wesentlich größeren Abstand haben als aufeinanderfolgende erste Zeitintervalle.

4. Verfahren nach einem der vorhergehenden Ansprüche, **dadurch gekennzeichnet, daß** die Nitrifikanten-Toxizität anhand eines Vergleichs des Abfallsgradienten des Sauerstoffgehaltes als Funktion der Zeit in der wäßrigen Lösung während eines Abschnitts des vierten Zeitintervalls mit dem Abfallsgradienten in reinem Wasser bestimmt wird.

5. Verfahren nach einem der vorhergehenden Ansprüche, **dadurch gekennzeichnet, daß** jeweils in vorgegebenen Zeitabständen zwischen dem ersten und dem nachfolgenden dritten Zeitintervall in einem fünften Zeitintervall eine Messung der Zeitabhängigkeit der Sauerstoffkonzentration in der wäßrigen Lösung ohne Nitrifikanten-Bakterien zur Gewinnung eines Korrekturwertes durchgeführt wird.

6. Verfahren nach Anspruch 4 und 5, **dadurch gekennzeichnet, daß** der Abfallsgradient des Sauerstoffgehaltes als Funktion der Zeit während eines Abschnittes des fünften Zeitintervalls als subtraktiver Korrekturwert für den im vierten Zeitintervall ermittelten Abfallsgradienten dient.

7. Verfahren nach einem der vorhergehenden Ansprüche, **dadurch gekennzeichnet, daß** in dem Kulturgefäß die Bakteriendichte der Nitrifikanten-Bakterienkultur und das Gesamtvolumen aus Nährlösung und Bakterienkultur jeweils auf einen konstanten Wert geregelt werden.

8. Verfahren nach Anspruch 5, **dadurch gekennzeichnet, daß** in dem Kulturgefäß kontinuierlich die Lichtstreuung gemessen und in Abhängigkeit vom Lichtstreu-Meßwert wahlweise gesteuert frische Nährlösung und/oder eine Verdünnungslösung dem Kulturgefäß zugeführt und ein dem zugeführten Volumen gleiches Volumen an Nährlösungs-Kultur-Mischung aus dem Kulturgefäß abgeleitet wird.

9. Verfahren nach einem der vorhergehenden Ansprüche, **dadurch gekennzeichnet, daß** als Nitrifikanten-Bakterien solche der Gattung(en) Nitrosomonas und/oder Nitrobacter eingesetzt werden.

10. Verfahren nach einem der vorhergehenden Ansprüche, **dadurch gekennzeichnet, daß** in dem Kulturgefäß kontinuierlich der pH-Wert gemessen und in Abhängigkeit vom pH-Meßwert wahlweise gesteuert ein pH-Regulans, insbesondere eine Base, dem Kulturgefäß zugeführt wird, um den pH-Wert, vorzugsweise bei einem Wert im Bereich zwischen 7,0 und 8,5, konstantzuhalten.

11. Verfahren nach einem der vorhergehenden Ansprüche, **dadurch gekennzeichnet, daß** als Nährlösung eine wäßrige Lösung mineralischer Komponenten mit einem Ammoniumsalz und/oder gelöstem Ammoniak und/oder Harnstoff als Stickstoffquelle und Hydrogencarbonat und/oder Carbonat und/oder gelöstem CO₂-Gas als Kohlenstoffquelle eingesetzt wird, die als Selektivnährlösung für ausgewählte Nitrifikanten-Bakterien wirkt.

12. Verfahren nach einem der vorhergehenden Ansprüche, **dadurch gekennzeichnet, daß** es bei einer im wesentlichen konstantgehaltenen Temperatur im Bereich zwischen 20°C und 40°C, bevorzugt zwischen 25°C und 30°C, duchgeführt wird.

13. Vorrichtung zur Durchführung des Verfahrens nach einem der vorhergehenden Ansprüche, **dadurch gekennzeichnet, daß** eine mit einem Zeitgeber (126) verbundene mehrkanalige Ablaufsteuerung (105; 105') vorgesehen ist, die jeweils nach Ablauf der vorbestimmten Zeitintervalle Steuersignale an Stellmittel (111, 118, 119, 120, 124; 111A, 111B, 118A, 118B, 120A, 120B, 124A, 124B) zum Öffnen bzw. Schließen ausgewählter mit der Meßzelle bzw. den Meßzellen verbundenen Leitungen und/oder an in diesen Leitungen angeordnete Einsatzstoff-Fördermittel (112, 117, 125; 112A, 112B, 117A, 117B) zu deren Aktivierung sowie an eine Sauerstoffgehalts-Auswertungseinrichtung (104) ausgibt.

14. Vorrichtung nach Anspruch 13, **dadurch gekennzeichnet, daß** in einem Kulturgefäß (101) eine Anordnung (115) zur Erfassung der Lichtstreuung und ein pH-Wert-Sensor (114) angeordnet sind, die mit einer Kulturbedingungs-Auswertungseinrichtung (128) verbunden sind, welche ihrerseits mit einem Dateneingang der Ablaufsteuerung verbunden ist und dieser Datensignale zuführt, die in Steuersignale für ein Nährlösungs-Absperrventil (106) und/oder eine Nährlösungs-Fördereinrichtung (107) und ein pH-Regulans-Absperrventil und/oder eine pH-Regulans-Fördereinrichtung (109) umgesetzt werden.

15. Vorrichtung nach Anspruch 13 oder 14, **dadurch gekennzeichnet, daß** an einer Zuflußleitung zur Meßzelle, mit dieser mittels durch Steuersignale der Ablaufsteuerung betätigter Absperrventile (119, 120; 120A, 120B) jeweils wahlweise verbindbar, ein in eine die wäßrige Lösung führende Leitung ragendes Probenahmeröhrchen (121a), ein Wasserleitungsanschluß (122) und ein Reinigungslösungsbehälter (123) vorgesehen sind.

16. Vorrichtung nach Anspruch 15, **dadurch gekennzeichnet, daß** an der Zuflußleitung zur Meßzelle Mittel (118; 118A) zur Einspeisung von Luft oder Sauerstoff vorgesehen sind, die vorzugsweise durch ein Steuersignal der Ablaufsteuerung wahlweise betätigbar sind.

17. Vorrichtung nach einem der Ansprüche 13 bis 16, **dadurch gekennzeichnet, daß** die Meßzelle einen durch die Ablaufsteuerung steuerbaren Thermostaten (116) aufweist.

18. Vorrichtung nach einem der Ansprüche 13 bis 17, **dadurch gekennzeichnet, daß** eine erste und eine zweite Meßzelle (102A, 102B) vorgesehen sind, wobei die erste Meßzelle mit einer die wäßrige Lösung führenden Leitung verbunden ist und die zweite Meßzelle mit dieser Leitung nicht verbunden ist.

19. Vorrichtung nach einem der vorhergehenden Ansprüche, **dadurch gekennzeichnet, daß** eine mindestens mittelbar mit dem Ausgang der Sauerstoffgehalts-Auswertungseinrichtung (104) verbundene Prozeßsteuereinrichtung (201) zur Ausgabe von Steuersignalen an Stellglieder (202 bis 204), insbesondere Schieber-Stelleinrichtungen, in einer Wasserversorgungs- oder Abwasserentsorgungsanlage (200) vorgesehen ist.

## Claims

1. A method for determining the nitrifying bacteria toxicity of an aqueous solution, in particular water or wastewater, by determining the time dependency of the oxygen content of a nitrifying bacteria culture, mixed with the previously aerated aqueous solution, in a closed measuring cell, in which the cultivation of the nitrifying bacteria culture is done in a nutrient solution in a separate culture vessel,
**characterized in that**
- an inlet to the measurement cell is periodically connected for a first time period to a line carrying the aqueous solution and after a predetermined number of first time periods or at predetermined times it is connected for a second time period to a line carrying pure water, wherein between the time periods, the measurement cell is evacuated via a drain, and at least for the majority of the first time period the drain is kept open in order to flush the measurement cell, and then the measurement cell is filled up to a predetermined level with, or with a predetermined quantity of, aerated aqueous solution or aerated pure water;
- after being filled with aqueous solution or pure water, the measurement cell is connected for a third time period to the culture vessel and is supplied with a predetermined quantity of the nitrifying bacteria culture; and
- after each third time period in the measurement cell, with the inlet and drain closed, in each case for a fourth time period a measurement of the time dependency of the oxygen concentration is carried out in alternation on mixtures of aqueous solution or pure water with the nitrifying bacteria culture.

2. A method for determining the nitrifying bacteria toxicity of an aqueous solution, in particular water or wastewater, by determining the time dependency of the oxygen content of a nitrifying bacteria culture, mixed with the previously aerated aqueous solution, in a closed measuring cell, in which the cultivation of the nitrifying bacteria culture is done in a nutrient solution in a separate culture vessel, **characterized in that**
- periodically for one first time period each, an inlet of the first measurement cell is connected to a line carrying the aqueous solution, and for a second time period an inlet of the second measurement cell is connected to a line carrying pure water, wherein between the first and second time periods the measurement cells are evacuated via a drain, and for at least the majority of the first time period the drain of the first measurement cell is kept open in order to flush the first measurement cell, and then the measurement cells are filled up to a predetermined, identical level with, or with a predetermined, identical quantity of, aerated aqueous solution or aerated pure water;
- after being filled with aqueous solution or pure water, the measurement cells are each connected for a third time period to the culture vessel and are supplied with a predetermined quantity of the nitrifying bacteria culture; and
- after each third time period in each of the measurement cells, with the inlet and drain closed, in each case for a fourth time period a measurement of the time dependency of the oxygen concentration is carried out in alternation on mixtures of aqueous solution or pure water with the nitrifying bacteria culture.

3. The method of claim 2, **characterized in that** successive second time periods are spaced substantially farther apart than successive first time periods.

4. The method of one of the preceding claims, **characterized in that** the toxicity for the nitrifying bacteria is determined from a comparison of the gradient of the decay in the oxygen content, as a function of time, in the aqueous solution during a portion of the fourth time period with the gradient of the decay in pure water.

5. The method of one of the preceding claims, **characterized in that** at predetermined time intervals, between a plurality of measurements each with the nitrifying bacteria culture, a measurement of the time dependency of the oxygen concentration in the aqueous solution without nitrifying bacteria culture is performed, to obtain a correction value.

6. The method of claim 4 and 5, **characterized in that** the gradient of the decay of the oxygen content as a function of time during a portion of a fifth time period serves as a subtractive correction value for the gradient of the drop ascertained in the fourth time period.

7. The method of one of the preceding claims, **characterized in that** in the culture vessel, the bacterial density of the nitrifying bacteria culture and the total volume of the nutrient solution in the bacteria culture are each regulated to a constant value.

8. The method of claim 5, **characterized in that** in the culture vessel, the light scattering is measured continuously, and fresh nutrient solution and/or a dilution solution is added to the culture vessel, selectively controlled as a function of the measured light scattering value, and a volume of nutrient solution and culture mixture equal to the supplied volume is drawn off from the culture vessel.

9. The method of one of the preceding claims, **characterized in that** as the nitrifying bacteria, those of the genus or genera Nitrosomonas and/or Nitrobacter are used.

10. The method of one of the preceding claims, **characterized in that** the pH value is measured continuously in the culture vessel, and a pH regulator, in particular a base, is supplied to the culture vessel, selectively controlled as a function of the measured pH value, in order to keep the pH value constant, preferably at a value in the range between 7.0 and 8.5.

11. The method of one of the preceding claims, **characterized in that** as the nutrient solution, an aqueous solution of mineral components with an ammonium salt and/or dissolved ammonia and/or urea as a nitrogen source and hydrogen carbonate and/or carbonate and/or dissolved CO₂ gas as a carbon source is used, which acts as a selected nutrient solution for selected nitrifying bacteria.

12. The method of one of the preceding claims, **characterized in that** it is performed at a temperature kept substantially constant in the range between 20°C and 40°C, preferably between 25°C and 30°C.

13. An apparatus for performing the method of one of the preceding claims, **characterized in that** a multichannel sequence controller (105; 105') connected to a timer (126) is provided, which in each case after the predetermined time periods have elapsed outputs control signals to adjusting means (111,; 118, 119, 120, 124; 111A, 111B, 118A, 118B, 120A, 120B, 124A, 124B) for opening or closing selected lines, communicating with the measurement cell or measurement cells, and/or to starting material feed means (112, 117, ,125; 112A, 112B, 117A, 117B), disposed in these lines, for their activation, and to an oxygen content evaluation device (104).

14. The apparatus of claim 13, **characterized in that** disposed in a culture vessel (101) are an arrangement (115) for detecting light scattering and a pH sensor (114) , which are connected to a culture condition evaluation device (128), which in turn is connected to a data input of the sequence controller and delivers data signals to it, which are converted into control signals for a nutrient solution shutoff valve (106) and/or a nutrient solution feed device (107) and a pH regulating shutoff valve and/or a pH regulator feed device (109).

15. The apparatus of claim 13 or 14, **characterized in that** a sampling tubule (121a), protruding into a line carrying the aqueous solution, a water line connection (122) and a cleaning solution container (123) are provided in an inlet line to the measurement cell and are each selectively connectable to the measurement cell by means of shutoff valves (119, 120; 120A, 120B) actuated by means of control signals of the sequence controller.

16. The apparatus of claim 15, **characterized in that** means (118; 118A) for feeding in air or oxygen are provided on the inlet line to the measurement cell and are preferably actuable selectively by a control signal of the sequence controller.

17. The apparatus of one of claims 13 to 16, **characterized in that** the measurement cell has a thermostat (116) that is controllable by the sequence controller.

18. The apparatus of one of claims 13 to 17, **characterized in that** a first and a second measurement cell (102A, 102B) are provided, the first measurement cell being connected to a line carrying the aqueous solution and the second measurement cell not being connected to that line.

19. The apparatus of one of the preceding claims, **characterized in that** a process controller (201), communicating at least indirectly with the outlet of the oxygen content evaluation device (104), is provided for outputting control signals to actuators (202-204), in particular slide positioners, in a water supply or wastewater disposal plant (200).

## Revendications

1. Procédé pour la détermination de la toxicité des bactéries nitriques d'une solution aqueuse, en particulier d'eau ou d'eaux usées, par la détermination de la dépendance temporelle du contenu d'oxygène d'une culture de bactéries nitriques présente dans une cellule de mesure fermée, et mélangée avec la solution aqueuse aérée au préalable, l'élevage de la culture de bactéries nitriques dans un bouillon de culture dans un récipient séparé constituant une partie du procédé, **caractérisé en ce que**
- une conduite d'amenée de la cellule de mesure est périodiquement mise en communication pour un premier intervalle de temps avec une conduite menant la solution aqueuse, et après un nombre prédéterminé de premiers intervalles de temps, ou à des moments prédéterminés pour un deuxième intervalle, est mise en communication avec une conduite menant de l'eau pure, la cellule de mesure étant vidée entre les intervalles de temps par une conduite d'écoulement, et la conduite d'écoulement étant maintenue ouverte pour la plus grande partie du premier intervalle de temps afin de rincer la cellule de mesure, et la cellule de mesure étant ensuite remplie jusqu'à un niveau prédéterminé ou avec une quantité prédéterminée de solution aqueuse aérée ou de l'eau pure aérée,
- la cellule de mesure, après avoir été remplie de solution aqueuse ou d'eau pure, est chaque fois mise en communication pour un troisième intervalle de temps avec le récipient de culture, et est alimentée, d'une quantité prédéterminée de la culture de bactéries nitriques, et
- après chaque troisième intervalle de temps, une mesure de la dépendance temporelle de la concentration d'oxygène est effectuée dans la cellule de mesure de manière alternante sur les mélanges de solution aqueuse ou d'eau pure et la culture des bactéries nitriques, la conduite d'amenée et d'écoulement étant chaque fois fermée pour un quatrième intervalle de temps.

2. Procédé pour la détermination de la toxicité des bactéries nitriques d'une solution aqueuse, en particulier d'eau ou d'eaux usées, par la détermination de la dépendance temporelle du contenu d'oxygène d'une culture de bactéries nitriques présente dans une cellule de mesure fermée, et mélangée avec la solution aqueuse aérée au préalable, l'élevage de la culture de bactéries nitriques dans un bouillon de culture dans un récipient séparé constituant une partie du procédé, **caractérise en ce que**
- périodiquement pour chacun des premiers intervalles de temps, une conduite d'amenée d'une première cellule de mesure, et pour un deuxième intervalle de temps, une conduite d'amenée d'une deuxième cellule de mesure qui est identique à la première cellule de mesure en termes de la technologie des procédés, est mise en communication avec une conduite menant de l'eau pure, les cellules de mesure, entre les premiers et deuxièmes intervalles de temps, étant vidées par une conduite d'écoulement et étant maintenues ouvertes au moins pour la plus grande partie du premier intervalle de temps afin de rincer la première cellule de mesure, et les cellules de mesure étant ensuite remplies jusqu'à un niveau prédéterminé ou avec une quantité prédéterminée identique de solution aqueuse aérée ou de l'eau pure aérée,
- les cellules de mesure, après avoir été remplies de solution aqueuse ou d'eau pure, sont chaque fois mise en communication pour un troisième intervalle de temps avec le récipient de culture, et sont alimentées d'une quantité prédéterminée de la culture de bactéries nitriques, et
- après le troisième intervalle de temps, une mesure de la dépendance temporelle de la concentration d'oxygène est effectuée dans les cellules de mesure de manière alternante sur les mélanges de solution aqueuse ou d'eau pure et la culture de bactéries nitriques, la conduite d'amenée et d'écoulement étant chaque fois fermée pour un quatrième intervalle de temps.

3. Procédé selon la revendication 2, **caractérisé en ce que** les deuxièmes intervalles de temps successifs ont un écart considérablement plus grand que les premiers intervalles de temps successifs.

4. Procédé selon l'une quelconque des revendications précédentes, **caractérisé en ce que** la toxicité des bactéries nitriques est déterminée au moyen d'une comparaison du gradient descendant du contenu d'oxygène en tant que fonction du temps dans la solution aqueuse pendant une période du quatrième intervalle de temps avec le gradient descendant dans de l'eau pure.

5. Procédé selon l'une quelconque des revendications précédentes, **caractérisé en ce que** chaque fois, dans des intervalles de temps prédéterminés entre le premier et le troisième intervalle successif, une mesure de la dépendance temporelle de la concentration d'oxygène dans la solution aqueuse sans bactéries nitriques est effectuée dans un cinquième intervalle de temps pour obtenir une valeur corrective.

6. Procédé selon la revendication 4 et 5, **caractérisé en ce que** le gradient descendant du contenu d'oxygène en tant que fonction du temps pendant une période du cinquième intervalle de temps sert comme valeur corrective et soustractive pour le gradient descendant déterminé dans le quatrième intervalle de temps.

7. Procédé selon l'une quelconque des revendications précédentes, **caractérisé en ce que** dans le récipient de culture, la densité des bactéries de la culture de bactéries nitriques et le volume total du bouillon de culture et de la culture de bactéries sont chacun réglés à une valeur constante.

8. Procédé selon la revendication 5, **caractérisé en ce que** la dispersion de la lumière est continuellement mesurée dans le récipient de culture, et un nouveau bouillon de culture et/ou une solution de dilution est introduit dans le récipient de culture de manière sélectivement réglée en dépendance de la valeur mesurée de la dispersion de la lumière, et un volume du mélange bouillon-culture égal au volume introduit est évacué du récipient de culture.

9. Procédé selon l'une quelconque des revendications précédentes, **caractérisé en ce que** comme bactéries nitriques sont utilisées celles de l'espèce/des espèces *Nitrosomonas* et/ou *Nitrobacter.*

10. Procédé selon l'une quelconque des revendications précédentes, **caractérisé en ce que** la valeur pH est continuellement mesurée dans le récipient de culture et que, en dépendance de la valeur pH, un régulateur pH, en particulier une base, est introduit dans le récipient de culture de manière sélectivement réglée, pour maintenir la valeur pH à une valeur comprise de préférence entre 7,0 et 8,5.

11. Procédé selon l'une quelconque des revendications précédentes, **caractérisé en ce qu'**une solution aqueuse de composants minéraux est utilisée comme bouillon de culture avec un sel d'ammonium et/ou de l'ammoniaque dissout et/ou de l'urée en tant que source d'azote, et carbonate d'hydrogène et/ou carbonate et/ou gaz CO₂ dissout en tant que source de carbone, qui agit comme bouillon de culture sélectif pour les bactéries nitriques choisies.

12. Procédé selon l'une quelconque des revendications précédentes, **caractérisé en ce que** le procédé est réalisé à une température essentiellement maintenue constante et comprise entre 20°C et 40°C, de préférence entre 25°C et 30°C.

13. Dispositif pour réaliser le procédé selon l'une quelconque des revendications précédentes, **caractérisé en ce qu'**une commande séquentielle (105 ; 105') multi-canale reliée à un générateur de rythme (126) est prévue, qui, en tout cas après l'expiration des intervalles de temps prédéterminés, émet des signaux de commande vers des organes de réglage (111, 118, 119, 120, 124 ; 111A, 1118, 1118A, 118B, 120A, 1208, 124A, 124B) pour l'ouverture ou la fermeture de conduites choisies et reliées à la cellule de mesure ou les cellules de mesure et/ou vers des moyens de transport (112, 117,125 ; 112A, 112B, 117A,117B) des substances utilisées pour activer ceux-ci, aussi bien que vers un moyen de dépouillement (104) du contenu d'oxygène.

14. Procédé selon la revendication 13, **caractérisé en ce qu**'un arrangement (115) pour détecter la dispersion de la lumière et un détecteur (114) pour la valeur pH sont disposés dans un récipient de culture (101), qui sont reliés à une unité de dépouillement (128) des conditions de culture, qui elle-même est reliée à une entrée de données de la commande séquentielle et amène des signaux de données à celle-ci, qui seront convertis en signaux de commande pour une soupape d'arrêt (106) pour le bouillon de culture et/ou un moyen de transport (107) du bouillon de culture, et pour une soupape d'arrêt du régulateur pH et/ou pour un moyen de transport (109) du régulateur pH.

15. Procédé selon la revendication 13 ou 14, **caractérisé en ce qu'**un tube échantillonneur (121a) faisant saillie dans une conduite menant la solution aqueuse, un raccord (122) à la conduite d'eau, et un récipient (123) de solution détergente sont prévus sur une conduite d'amenée à la cellule de mesure, et qui peuvent être reliés à celle-ci chacun de manière sélective au moyen de soupapes d'arrêt (119, 120 : 120A, 120B) qui peuvent être actionnées par des signaux de commande de la commande séquentielle.

16. Procédé selon la revendication 15, **caractérisé en ce que** des moyens (118 ; 188A) pour l'alimentation d'air ou d'oxygène sont prévus sur la conduite d'amenée à la cellule de mesure, qui peuvent être actionnés de préférence par un signal de commande de la commande séquentielle de manière sélective.

17. Dispositif selon l'une quelconque des revendications 13 à 16, **caractérisé en ce que** la cellule de mesure comporte un thermostat (116) qui peut être commandé par la commande séquentielle.

18. Dispositif selon l'une quelconque des revendications 13 à 17, **caractérisé en ce qu**'une première et une deuxième cellule de mesure (102A, 102B) sont prévues, la première cellule de mesure étant reliée à la conduite menant la solution aqueuse, et la deuxième cellule de mesure n'étant pas reliée à cette conduite.

19. Dispositif selon l'une quelconque des revendications 13 à 17, **caractérisé en ce qu**'un moyen de commande de processus (201) au moins indirectement relié à la sortie du moyen de dépouillement (104) pour le contenu d'oxygène est prévu dans un système (200) de distribution d'eau ou d'évacuation des eaux usées, pour émettre des signaux de commande à des organes de réglage (202 à 204), en particulier des moyens de réglage pour des vannes d'arrêt.
